# EUROPEAN PATENT APPLICATION

(11) **EP 4 843 589 A1**
(43) Date of publication of application: **25.11.2026**
(21) Application number: 25741956.4
(22) Date of filing: 15.01.2025
(51) Int. Cl.: A61L 27/52, A61L 27/24, A61L 27/40, A61L 27/56, A61P 27/16

(54) **MEDICAL MATERIAL**

(30) Priority: 18.01.2024 JP 2024005973
(71) Applicant: National University Corporation Kagawa University, Takamatsu-shi, Kagawa 760-8521 (JP)
(72) Inventor: MIYASHITA Takenori, Kita-gun, Kagawa 761-0793 (JP)
(74) Representative: Praxi Intellectual Property Lugano
(86) International application number: PCT/JP2025/000990
(87) International publication number: WO 2025/154731

(57) **Abstract**

A medical material includes a hydrogel sheet including a base portion, an openable and closable grasping portion, and a slit for separating a part of the grasping portion from the base portion.

## Description

### TECHNICAL FIELD

The present invention relates to a medical material. Priority is claimed on Japanese Patent Application No. 2024-005973, filed January 18, 2024, the content of which is incorporated herein by reference.

### BACKGROUND ART

There are 100,000 patients per year in Japan alone with tympanic membrane perforation caused by otitis media or trauma. Hearing loss is one of three major risk factors for dementia, and treatment of tympanic membrane perforation and hearing loss is important also for preventing dementia. Since otorrhea damages hearing aids, hearing aids cannot be used for ears with tympanic membrane perforation, which causes not only an increased risk of dementia in patients with tympanic membrane perforation but also a significant decrease in QOL.

In tympanic membrane regeneration surgery, it has been necessary to reliably attach and bring a dense collagen membrane into close contact with a middle ear side that is an inner side of the tympanic membrane. However, since a conventional dense collagen membrane is difficult to grasp, there is a possibility that the dense collagen membrane in the related art is drawn in by suction and lost, or is misplaced into an unintended site.

In addition, during surgery for otitis media, when a middle ear mucosal defect portion (exposed bone surface portion) becomes extensive, regeneration of middle ear mucosa becomes insufficient, and complications such as adhesion occur. At present, however, there is no medical material that covers a middle ear mucosal defect portion from which a large amount of exudate seeps and promotes mucosal regeneration.

In order to improve such uncertainty and poor operability, the inventors developed a dried vitrigel membrane body integrally including a grasping thread portion and a face portion (see, for example, Patent Document 1).

### Citation List

### Patent Documents

Patent Document 1: Japanese Patent No. 7350238

### SUMMARY OF INVENTION

### Technical Problem

By using the dried vitrigel membrane body disclosed in Patent Document 1, handling is significantly improved. However, production of such a dried vitrigel membrane body is difficult, and the grasping thread portion may become an obstacle. Therefore, there is room for further improvement.

The present invention has been made in view of the above circumstances, and provides a medical material that is easy to produce and has excellent handling performance.

### Solution to Problem

The present invention includes the following aspects.
[1] A medical material including a hydrogel sheet that includes a base portion, an openable and closable grasping portion, and a slit for separating a part of the grasping portion from the base portion.
[2] The medical material according to [1], in which the grasping portion is openable and closable in a direction intersecting a sheet surface of the hydrogel sheet.
[3] The medical material according to [1], in which the grasping portion is surrounded by the base portion.
[4] The medical material according to [1], in which, in a cross-sectional view of the sheet taken along a direction in which the grasping portion extends from the base portion, the slit is formed obliquely with respect to the sheet surface.
[5] The medical material according to Claim 1, wherein the medical material is for use in membrane regeneration, mucosal regeneration, or epithelial regeneration.
[6] The medical material according to [1], in which the slit has a U-shape.
[7] The medical material according to [1], in which the weight per unit area of the hydrogel sheet is 0.1 mg/cm² to 50 mg/cm².

### Advantageous Effects of Invention

According to the present invention, it is possible to provide a medical material that is easy to produce and has excellent handling performance.

### BRIEF DESCRIPTION OF DRAWINGS

[FIG. 1] A front view of a medical material 100 according to the present embodiment.
[FIG. 2] A cross-sectional view of the medical material 100 taken along a line II-II in FIG. 1.
[FIG. 3] A cross-sectional view of the medical material 100 taken along a line III-III in FIG. 1.
[FIG. 4] A front view showing an example of the medical material according to the present embodiment.
[FIG. 5] A front view of a medical material having a through hole.
[FIG. 6] A cross-sectional view of the medical material taken along a line VI-VI in FIG. 5.
[FIG. 7] A front view of a medical material having a dimple.
[FIG. 8] A cross-sectional view of the medical material taken along a line VIII-VIII in FIG. 7.
[FIGS. 9A to 9E] Photographs showing steps of confirming a function of a grasping structure using Parafilm.
[FIG. 10] A photograph showing confirmation of a function of a grasping structure using a vitrigel membrane.
[FIG. 11] A photograph of a wound site 4 days after implantation of a vitrigel membrane with a grasping portion or Neoveil (registered trademark).
[FIG. 12] A photograph showing a result of HE staining of a tissue section in FIG. 11.
[FIG. 13] A photograph of a wound site 7 days after implantation of a vitrigel membrane or an existing collagen sponge.
[FIG. 14] A graph showing measurement results of a tympanic membrane perforation size in FIG. 13.

### DESCRIPTION OF EMBODIMENTS

Hereinafter, embodiments of the present invention will be described in detail with reference to the drawings as appropriate. The dimensional ratios in the respective drawings may be exaggerated for the sake of explanation, and do not necessarily match the actual dimensional ratios.

### <<Medical material>>

In one embodiment, the present invention provides a medical material including a hydrogel sheet that includes a base portion, an openable and closable grasping portion, and a slit for separating a part of the grasping portion from the base portion.

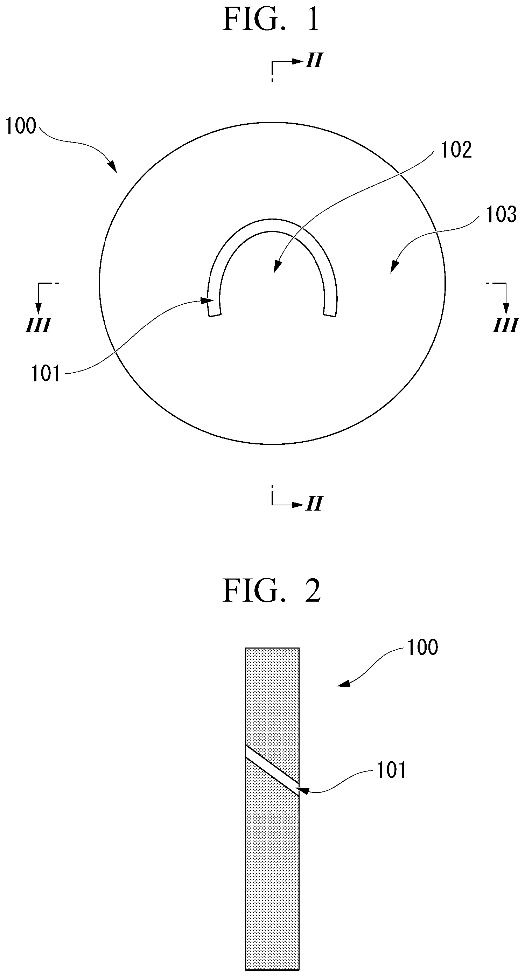

FIG. 1 is a front view of a medical material 100. The medical material 100 includes, on a hydrogel sheet, a base portion 103 and an openable and closable grasping portion 102. A part of the grasping portion 102 is separated from the base portion 103 by a slit 101 provided in the hydrogel sheet. The grasping portion 102 is defined by the separation.

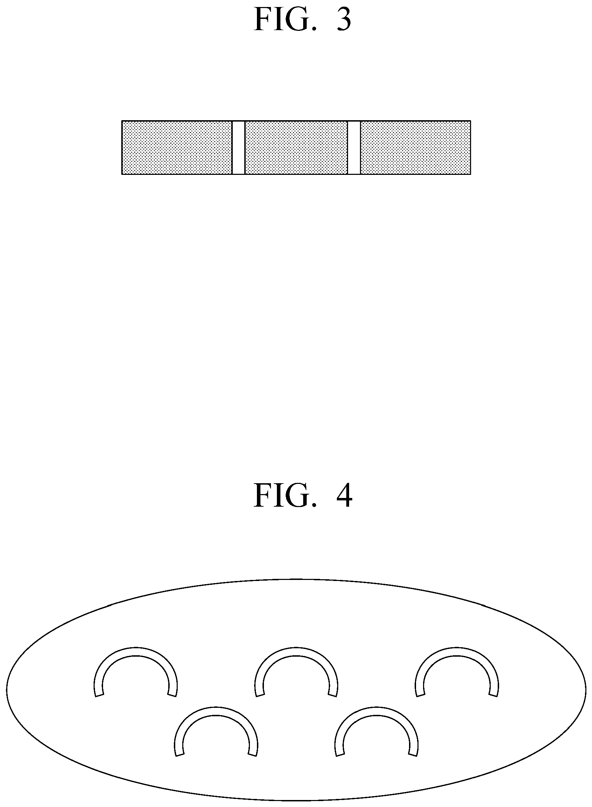

FIG. 2 is a cross-sectional view of the medical material 100 taken along a line II-II in FIG. 1. In a cross-sectional view of the hydrogel sheet taken along a direction in which the grasping portion 102 extends from the base portion 103, the slit 101 is formed obliquely with respect to a surface of the hydrogel sheet. In the present embodiment, this configuration provides a strong connection between the grasping portion 102 and the base portion 103, thereby maintaining a grasping structure. In the present embodiment, because the slit is formed obliquely and slits on the front and back are shifted from each other, the grasping portion is automatically accommodated when grasping is released after the grasping portion is grasped. Furthermore, the slit portion is closed without leaving a gap in the slit, and the surfaces (both surfaces) become flat, so that no gap remains in the slit. That is, in the present embodiment, no step is formed in the regenerated cell sheet, and the regenerated cell sheet becomes smooth. FIG. 3 is a cross-sectional view of the medical material 100 taken along a line III-III in FIG. 1. In a cross-sectional view of the hydrogel sheet taken along a direction perpendicular to a direction in which the grasping portion 102 extends from the base portion 103, the slit 101 is formed perpendicular to a surface of the hydrogel sheet.

As shown in FIG. 1, the portion delineated by the slit 101 functions as the grasping portion 102. The grasping portion 102 is openable and closable in a direction intersecting a sheet surface of the hydrogel sheet. The grasping portion 102 is surrounded by the base portion 103 and has a flap structure that is openable and closable. The shape of the slit 101 is not particularly limited as long as the grasping portion 102 can be formed. Examples of the shape include a squared U-shape, a U-shape having an arcuate or semicircular configuration, and a triangular shape, with a U-shape being preferred. The slit 101 may also have a linear shape, such as a straight-line shape or a wavy-line shape, provided that the shape permits grasping.

A size of the grasping portion formed by the slit is not particularly limited, and for example, when the grasping portion has a U-shape, a diameter thereof is preferably 0.5 mm to 30 mm and more preferably 1.5 mm to 3.0 mm. For example, a grasping portion having such a diameter is formed by post-processing, such as pressing a 23G needle having a U-shaped tip. In this case, a width of the slit depends on a width of the needle, and is less than 1 mm.

The number of slits in the medical material according to the present embodiment is not particularly limited and may be varied depending on an area of the hydrogel sheet. When the medical material is for a human tympanic membrane or for animals, one slit is preferable, as shown in FIG. 1. When the medical material is for a human middle ear, a plurality of slits is preferable, as shown in FIG. 4.

Arrangement of the slits in the medical material according to the present embodiment may be appropriately adjusted for convenience in surgery or treatment. Since the hydrogel sheet is appropriately cut according to the shape of an affected part of a patient, the slits are preferably arranged evenly, as shown in FIG. 4, so that the biomedical material has a slit after being cut into any shape.

The shape of the hydrogel sheet in the medical material according to the present embodiment is not particularly limited. Polygons such as a triangle, a quadrangle (including a square, a rectangle, and a trapezoid), a pentagon, a hexagon, a heptagon, and an octagon; a circle, an ellipse, a substantially circular shape, an ellipse, a substantially elliptical shape, a semicircle, a sector shape, and the like are exemplary examples. The hydrogel sheet is appropriately cut into a shape and size according to the shape of the affected part. For example, for use in a human tympanic membrane, a circular medical material having a diameter of 8 mm is contemplated; and for use in a human middle ear, a circular medical material having a diameter of 3 cm is contemplated.

An area of the medical material according to the present embodiment may be appropriately set as necessary and may be, for example, 1 mm² to 400 cm², 20 mm² to 40 cm², or 80 mm² to 4 cm².

The medical material according to the present embodiment may have a flat surface, a curved surface, or an uneven surface, and may be selectively used according to the application. The thickness of the medical material according to the present embodiment may be appropriately set as necessary and may be, for example, 0.1 µm to 5 mm, 2 µm to 1 mm, or 20 µm to 400 µm. Furthermore, the thickness of the medical material according to the present embodiment may be uniform or non-uniform.

The weight per unit area of the medical material according to the present embodiment is preferably 0.1 mg/cm² to 50 mg/cm², and more preferably 0.5 mg/cm² to 5 mg/cm². As the hydrogel sheet becomes thinner, the hydrogel sheet becomes softer and more easily conforms to an uneven surface of a wound site. Moisture such as exudate also more easily passes through the hydrogel sheet, but the sheet becomes more likely to curl, making handling difficult. For middle ear mucosal regeneration, the above-described weight is preferably 0.7 mg/cm² or more, more preferably 1 mg/cm² or more, and still more preferably 1.4 mg/cm² or more. For tympanic membrane regeneration, the above-described weight is preferably 2 mg/cm² or more, more preferably 3 mg/cm² or more, and still more preferably 4 mg/cm² or more.

In contrast, as described later in Examples, a Neoveil sheet (registered trademark; PGA) has coarse fibers and thus cannot cover a wound surface, and mucosal regeneration is not promoted.

Since the medical material 100 has the grasping portion 102, surgical time can be significantly shortened, and the medical material can be easily fixed to a wound surface without being washed away by exudate. In addition, even when exudate is discharged from a wound site of a living body, the exudate can pass through an opening formed in the medical material 100 by the slit defining the grasping portion. Furthermore, it is confirmed by Examples described later that middle ear mucosal regeneration and tympanic membrane regeneration can be significantly promoted. Development of a novel medical material having such high exudate permeability and a grasping mechanism enables less invasive, reliable, and rapid reconstructive surgery or treatment.

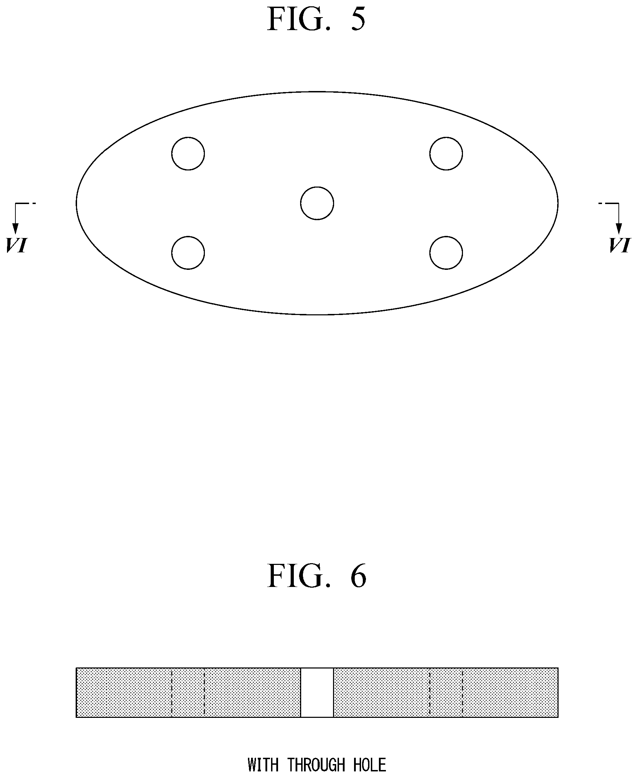

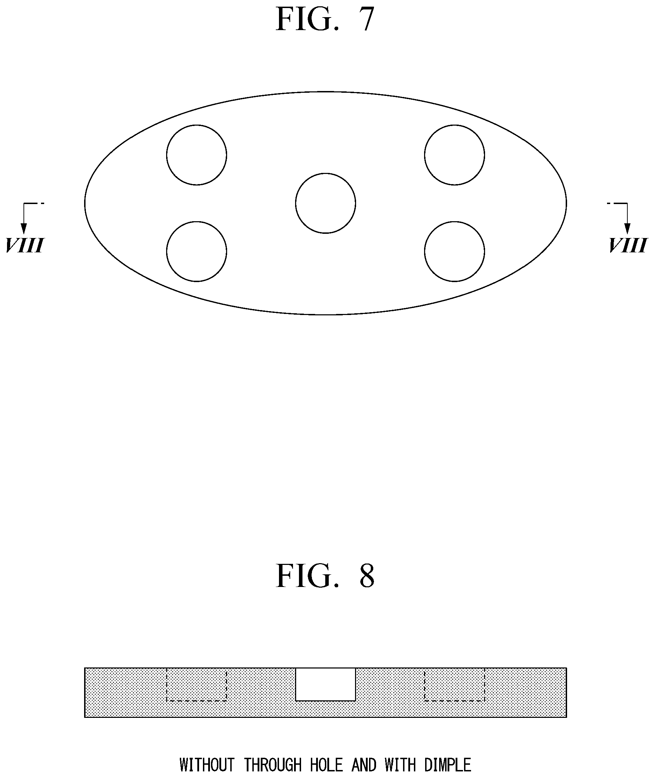

The medical material according to the present embodiment preferably has no through hole (perforation). When the medical material according to the present embodiment is implanted onto a middle ear bony wall, in a case where the medical material has a through hole as shown in FIGS. 5 and 6, the medical material can allow exudate from bone to pass through the through hole while conforming to and closely contacting unevenness of the middle ear bony wall. However, unevenness is formed in regenerated mucosal tissue. In order to increase exudate permeability, the medical material according to the present embodiment preferably has a dimple instead of the through hole, as shown in FIGS. 7 and 8.

In the present specification, the term "sol," which serves as a raw material for "hydrogel," means one in which colloidal particles of a dispersoid having a liquid as a dispersion medium (size: approximately 1 nm to several hundred nm) are composed particularly of a polymer compound. More specifically, aqueous solutions of natural polymer compounds or synthetic polymer compounds are exemplary examples of the sol. When crosslinks are introduced by chemical bonds into these polymer compounds and the polymer compounds form a network structure, the above-described sol transitions into the "hydrogel," which is a semi-solid substance holding a large amount of water in the network. That is, the term "hydrogel" means a gelled sol.

The sol serving as a raw material for the hydrogel may be any material having biocompatibility. A gelling extracellular matrix-derived component; natural polymer compounds such as fibrin, agar, agarose, and cellulose; and synthetic polymer compounds such as polyacrylamide, polyvinyl alcohol, polyethylene oxide, and poly(II-hydroxyethyl methacrylate)/polycaprolactone are exemplary examples of the sol.

Collagen (type I, type II, type III, type V, type XI, and the like), a basement membrane component (trade name: Matrigel) reconstituted from a mouse EHS tumor extract (including type IV collagen, laminin, heparan sulfate proteoglycan, and the like), glycosaminoglycan, hyaluronic acid, proteoglycan, gelatin, and the like are exemplary examples of the gelling extracellular matrix-derived component, but the gelling extracellular matrix-derived component is not limited thereto. A desired hydrogel can be produced by selecting components, such as salts, that are optimal for each gelation, concentrations of the components, pH, and the like. In addition, combining the raw materials also provides hydrogels that mimic various in vivo tissues.

Among these, the sol is preferably a gelling extracellular matrix-derived component, and more preferably collagen. In addition, native collagen and atelocollagen are exemplary examples of a more preferable raw material among collagens, and atelocollagen is still more preferable.

"Vitrigel" refers to a stable gel obtained by rehydrating a conventional hydrogel after vitrification, in which, after free water in the hydrogel is completely removed, partial removal of bound water is allowed to proceed. The gel has been named "Vitrigel (registered trademark)" by the present inventor.

In the present specification, a dried hydrogel body that is immediately after the vitrification step and has not undergone a rehydration step is simply referred to as "dried hydrogel body". A gel obtained through the rehydration step after the vitrification step is referred to separately as "vitrigel," and a dried body obtained by vitrifying the vitrigel is referred to as "dried vitrigel body". Accordingly, the "vitrigel" is a hydrated body.

In addition, in the present specification, when the term "vitrigel" is used, "(registered trademark)" may be omitted.

In the present specification, the concept of "hydrogel" includes "vitrigel".

In the present specification, the concept of "hydrogel sheet" includes "dried hydrogel sheet body".

### <Applications>

Because the medical material according to the present embodiment has an openable and closable grasping portion defined by the slit, the medical material is suitably used for, for example, surgery and the like, which require complicated manipulations until the medical material reaches an application site. Specifically, the medical material according to the present embodiment is suitably used when the medical material is applied to an inner wall of the esophagus through the esophagus, when the medical material is applied to the tympanic membrane through the external auditory canal, or when the medical material is fixed to various wound sites.

The medical material according to the present embodiment is preferably for use in membrane regeneration, mucosal regeneration, or epithelial regeneration. A middle ear, a tympanic membrane, an external auditory canal, a nasal cavity, a paranasal sinus, and the like are exemplary examples of an application site of the medical material according to the present embodiment. When the medical material according to the present embodiment is applied to a middle ear, a tympanic membrane, or an external auditory canal, the medical material according to the present embodiment is preferably for use in tympanic membrane regeneration or middle ear mucosal regeneration. In addition, the medical material according to the present embodiment is also used for oral mucosa, pharyngeal mucosa, laryngeal mucosa, gastrointestinal mucosa, or the like.

The medical material according to the present embodiment is suitably used for treatment of tympanic membrane perforation. In intractable otitis media, tympanic membrane perforation does not heal spontaneously, and fascia is usually used; however, this is highly invasive to the patient and the surgical procedure has been difficult. Because tympanic membrane tissue regenerates so as to project into air, the presence of a scaffold promotes regeneration. In addition, the properties of the tympanic membrane differ greatly between an epithelial layer (stratified squamous epithelium) and a mucosal layer (mucosa). The epithelial layer of the tympanic membrane constantly migrates from a central portion toward a peripheral portion and then toward an outer side of the external auditory canal at a speed of 0.07 mm/day. Therefore, when a scaffold is attached to the epithelial layer, that is, the outer side of the tympanic membrane, the scaffold is displaced at a speed of 0.07 mm/day. Accordingly, by using an underlay method in which the medical material according to the present embodiment is attached to the middle ear side, which does not migrate, the medical material according to the present embodiment can function as a scaffold for a long period. In animal experiments as well, tympanic membrane reconstruction needs to be performed using the underlay method in the same manner as in human surgery. In tympanic membrane reconstruction in tympanoplasty, such as endoscopic tympanoplasty, it is necessary to make a skin incision and harvest a material for tympanic membrane reconstruction, such as subcutaneous connective tissue, perichondrium, or cartilage. In contrast, the medical material according to the present embodiment enables tympanoplasty to be performed without making a skin incision.

In addition, when attached to a middle ear mucosal defect portion during middle ear surgery, the medical material according to the present embodiment functions as an absorbable artificial middle ear mucosa that promotes regeneration of the patient's own middle ear mucosa. The medical material according to the present embodiment, having the openable and closable grasping portion defined by the slit, is an optimal solution for middle ear mucosal regeneration treatment. The medical material according to the present embodiment is suitably used not only for chronic otitis media but also for tympanic membrane regeneration in adhesive otitis media and cholesteatomatous otitis media. The medical material can provide an innovative new treatment method even for intractable otitis media that cannot be fully addressed by tympanic membrane regeneration alone. The medical material according to the present embodiment is less invasive to a patient and can facilitate surgery.

### [Method for treating tympanic membrane perforation]

A method for treating tympanic membrane perforation according to the present embodiment includes: a step of grasping the grasping portion of the above-described medical material, and passing the medical material through a tympanic membrane perforation from an external ear side to a middle ear side; and a step of pulling the medical material that has passed through the tympanic membrane perforation from the middle-ear side toward the external-ear side and attaching a surface of the medical material to the middle-ear-side surface of the tympanic membrane.

### <<Manufacturing method>>

The medical material according to the present embodiment is manufactured by post-processing the hydrogel sheet, such as by pressing the hydrogel sheet with a needle having a U-shaped tip. The method described below is an exemplary example of the method for manufacturing the hydrogel sheet.

First, a sol is prepared. When the sol to be injected is a collagen sol, a collagen sol prepared using, for example, physiological saline, phosphate buffered saline (PBS), Hank's Balanced Salt Solution (HBSS), basal medium, serum-free medium, serum-containing medium, or the like may be used as the collagen sol having an optimal salt concentration. In addition, a pH of the solution during collagen gelation may be, for example, 6 to 8. In addition, the preparation of the collagen sol may be performed at, for example, approximately 4°C.

In particular, when a serum-free medium is used, it is possible to prevent substances contained in serum components of other animals and unsuitable for application to a wound site of a living body (for example, antigens, pathogenic factors, and the like) from being contained in the hydrogel. Therefore, a hydrogel membrane obtained using the serum-free medium is suitably used for medical purposes.

In addition, a concentration of the collagen sol for producing the hydrogel is preferably 0.1 to 1.0 mass%, and more preferably 0.2 to 0.6 mass%. When the concentration of the collagen sol is the above-described lower limit value or greater, gelation is not excessively weak, and when the concentration of the collagen sol is the above-described upper limit value or less, a hydrogel made of a uniform collagen gel can be obtained.

Next, the sol is left to stand to undergo gelation, whereby a hydrogel sheet is obtained. A temperature at which the sol is maintained can be appropriately adjusted according to the type of sol used. For example, when the sol is a collagen sol, the temperature at which the sol is maintained during gelation can be a temperature lower than a denaturation temperature of collagen, which depends on the animal species of the collagen used. In general, the gelation can be performed in several minutes to several hours by maintaining the sol at a temperature of 20°C or higher and 37°C or lower.

Next, the hydrogel sheet may be irradiated with ultraviolet rays. For irradiation with ultraviolet rays, a known ultraviolet irradiation device can be used. The irradiation energy is preferably such that the total irradiation amount per unit area is 0.1 mJ/cm² to 6000 mJ/cm², more preferably 10 mJ/cm² to 4000 mJ/cm², still more preferably 100 mJ/cm² to 3000 mJ/cm², and particularly preferably 200 mJ/cm² to 1500 mJ/cm².

When the total irradiation amount is within the above-described range, particularly favorable transparency and strength of the hydrogel sheet can be obtained. When the transparency of the hydrogel sheet interferes with operability, the ultraviolet irradiation step may be omitted.

When the total ultraviolet irradiation amount per unit area is the same, the transparency and strength of the hydrogel sheet can be further increased by dividing irradiation of the hydrogel sheet with ultraviolet rays into a plurality of irradiation operations and repeating the irradiation.

Next, the hydrogel sheet is dried. Drying the hydrogel sheet makes it possible to completely remove free water from the hydrogel sheet and to further advance partial removal of bound water. As the period of the vitrification step (a step of advancing partial removal of bound water after completely removing free water in the hydrogel sheet) is increased, a vitrigel sheet having excellent strength can be obtained when rehydrated.

Various drying methods may be used, such as air drying, drying in a sealed container (circulating air in the container and constantly supplying dry air), and drying in the presence of silica gel. Examples of the air-drying method include drying for 2 days in an incubator kept aseptic at 10°C and 40% humidity, and drying overnight at room temperature in an aseptic clean bench.

Through the above-described steps, a hydrogel sheet or a dried hydrogel sheet body is obtained. When the hydrogel sheet or the dried hydrogel sheet body is to be manufactured with an increased thickness, it is preferable to repeat a step of layering a sol on the hydrogel sheet, allowing the layered sol to stand to undergo gelation, and drying the layered hydrogel sheet.

A vitrigel sheet is obtained by hydrating the above-described dried hydrogel sheet body, and a dried vitrigel sheet body is obtained by drying the vitrigel sheet. Examples of the aqueous solution used for hydration include sterile water, physiological saline, and PBS. In the hydration, it is preferable to repeat hydration while appropriately replacing the aqueous solution.

### Examples

Hereinafter, the present invention will be described with reference to Examples, but the present invention is not limited to Examples.

### [Confirmation of function of grasping structure using Parafilm]

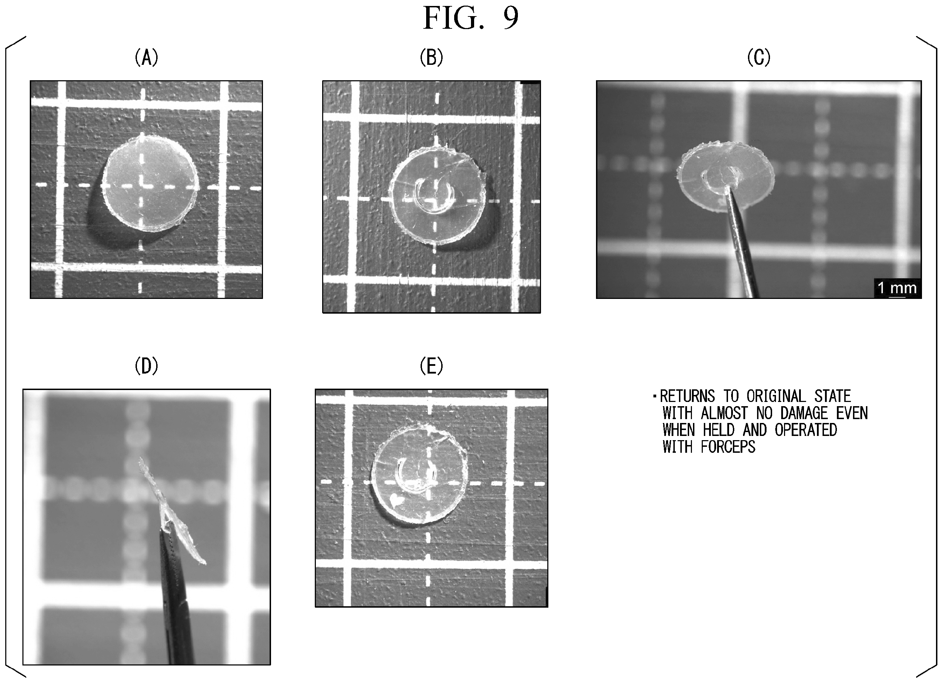

Parafilm was cut into a circular shape having a diameter of 6 mm (see FIG. 9A), and slit processing was performed using a slit-forming device (see FIG. 9B). As shown in FIGS. 9C and 9D, a grasping portion was formed by the slit processing and could be held with forceps. As shown in FIG. 9E, it was confirmed that, after the operation, the circular Parafilm had almost no damage and returned to its original state.

### [Production of vitrigel membrane with grasping portion]

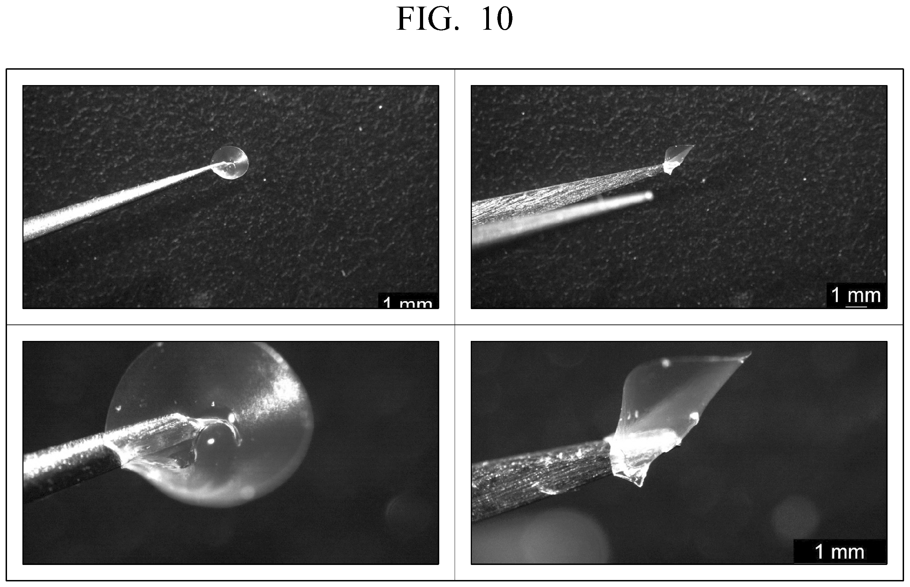

A revitrified dried atelocollagen vitrigel sheet body was cut into a circular shape having a diameter of 6 mm, and slit processing was performed using a slit-forming device. As shown in FIG. 10, a grasping portion was formed by the slit processing, and a vitrigel membrane with a grasping portion was obtained. The vitrigel membrane with a grasping portion could be held with forceps.

### [Rat middle ear mucosal regeneration experiment]

Endoscopic otologic surgery was performed on a temporal bone surgery model using the above-described vitrigel membrane with a grasping portion as a collagen membrane for tympanic membrane reconstruction. The conditions of the surgery were as follows. As a comparative control, an existing PGA sheet (Neoveil (registered trademark); circular shape having a diameter of 6 mm) was used.
Perforation size: diameter of 4 mm
Vitrigel membrane: size diameter of 6 mm, 1.4 mg/cm²
Endoscope used: Olympus rigid endoscope having a diameter of 4 mm
Ear alligator forceps
Slightly curved Rosen needle

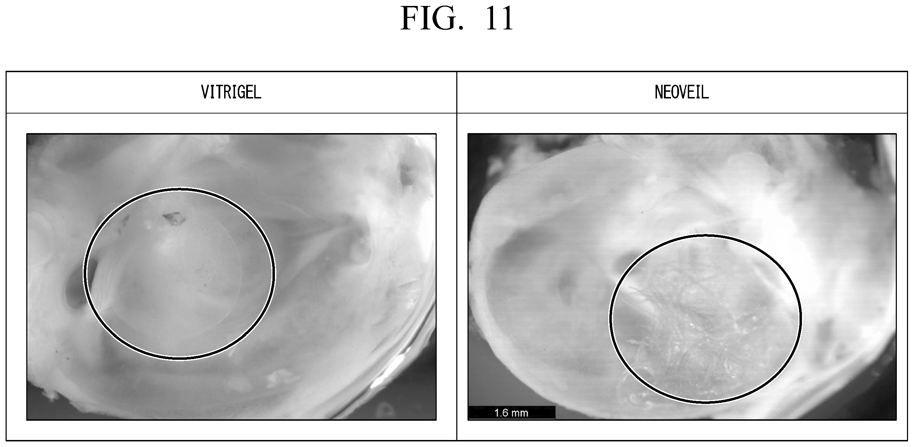

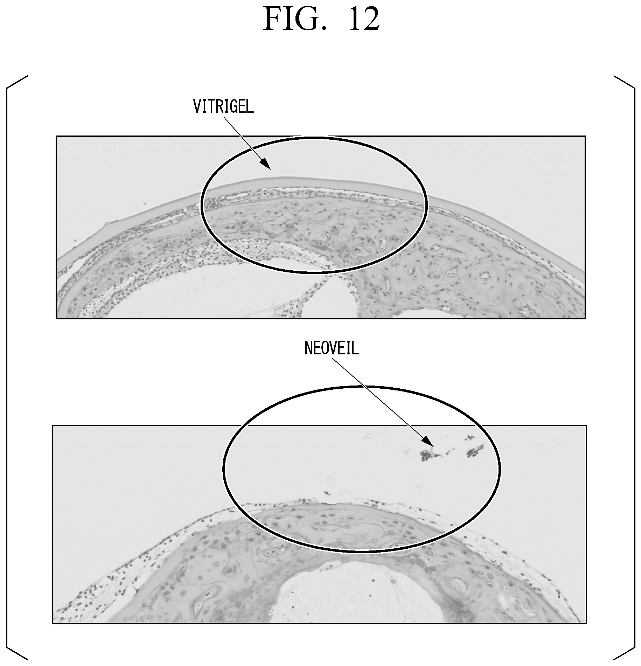

FIG. 11 shows a photograph of a wound site 4 days after implantation. The vitrigel membrane with a grasping portion conformed to the object to be covered. In contrast, Neoveil did not cover the wound surface because the fibers were coarse. FIG. 12 further shows the results of HE staining of the tissue section. In the Neoveil-treated rat, a mucosal defect was observed, and it was confirmed that mucosal cells were not aligned. In contrast, in the rat treated with the vitrigel membrane with a grasping portion, it was confirmed that mucosal cells were neatly aligned.

It was confirmed that Neoveil did not sufficiently promote middle ear mucosal regeneration because the fibers were coarse and the wound surface could not be covered. On the other hand, it was confirmed that the vitrigel membrane with a grasping portion covered and protected the middle ear mucosal defect portion and promoted middle ear mucosal regeneration. It was confirmed that providing a slit in the vitrigel allowed exudate from the middle ear bone wall to pass through the vitrigel and allowed the vitrigel to continue adhering to the middle ear bone surface (mucosal defect surface). It was confirmed that the slit did not adversely affect mucosal cell proliferation.

### [Rat tympanic membrane perforation regeneration experiment]

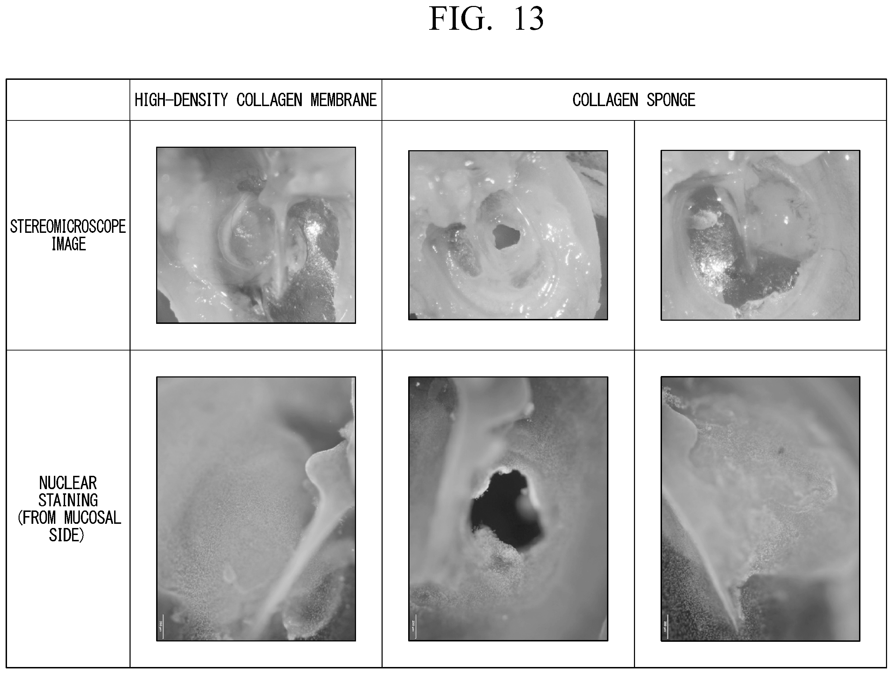

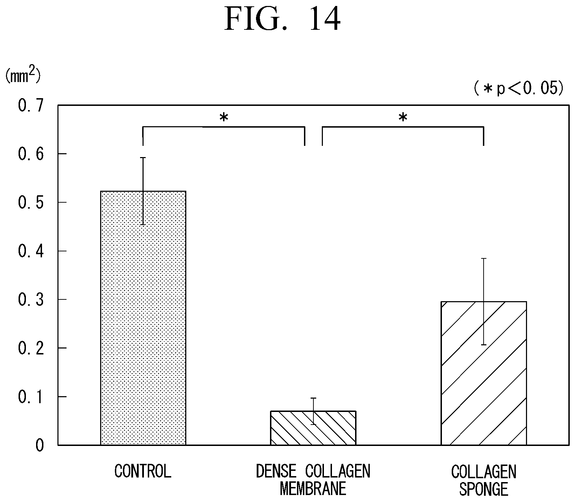

Using a tympanic membrane perforation model rat in which tympanic membrane perforation had been produced by heat, tympanic membrane reconstruction was performed by the underlay method using the vitrigel membrane, and the effectiveness was examined. Tympanic membrane reconstruction was similarly performed by the underlay method using an existing collagen sponge, and the effectiveness was comparatively examined. FIGS. 13 and 14 show results of the tympanic membrane perforation size on day 7 after treatment. When the collagen sponge was used, the tympanic membrane perforation could not be closed, and granulation tissue was observed. In contrast, the tympanic membrane perforation was closed by the vitrigel membrane.

### [Investigation of thickness of vitrigel membrane with grasping portion]

When the vitrigel membrane with a grasping portion used in middle ear surgery is too thin, the vitrigel membrane with a grasping portion tends to fold or bend; and when the vitrigel membrane with a grasping portion is too thick, the vitrigel membrane with a grasping portion is less likely to closely adhere to a middle ear bone wall. The vitrigel membrane with a grasping portion needs to allow exudate to pass through. Therefore, the thickness of the vitrigel membrane with a grasping portion was examined. Dense collagen sheets used in the examination are shown below.
Diameter of atelocollagen vitrigel: 1.5 mm
1.4 mg/cm² (for tympanic membrane experiment)
0.7 mg/cm²
0.5 mg/cm²

When the vitrigel membrane with a grasping portion was thin, the membrane tended to adhere to a petri dish under the influence of static electricity, and was susceptible to the surface tension of an aqueous solution. On the other hand, when the vitrigel membrane with a grasping portion was thin, the aqueous solution quickly permeated the membrane.

The 0.5 mg/cm² vitrigel membrane with a grasping portion tended to become transparent and difficult to see when placed in physiological saline, and curled into a ball-like shape.

The 0.7 mg/cm² vitrigel membrane with a grasping portion spread when placed in physiological saline, and was easy to handle when an applicator was used.

Next, UV irradiation of the vitrigel membrane with a grasping portion was examined. The vitrigel membranes with a grasping portion used in the examination are shown below.
Diameter of atelocollagen vitrigel: 1.5 mm
0.7 mg/cm², no UV irradiation
0.7 mg/cm², UV: 80 mJ/cm²
0.7 mg/cm², UV: 800 mJ/cm²
0.5 mg/cm², no UV irradiation
0.5 mg/cm², UV: 80 mJ/cm²
0.5 mg/cm², UV: 800 mJ/cm²

The 0.5 mg/cm² vitrigel membrane with a grasping portion irradiated with UV at 800 mJ/cm² became more transparent. At a diameter of 1.5 mm, the hardness also did not change greatly compared with the membrane without UV irradiation.

Regarding the effect of UV irradiation on the 0.7 mg/cm² vitrigel membrane with a grasping portion, the membrane without UV irradiation had higher flexibility and properties somewhat close to jelly. The membrane easily conformed to irregularities and was easy to place on an applicator. The water content also appeared to be higher without UV irradiation, and the membrane changed to be slightly thicker and was easy to use.

The 0.7 mg/cm² membrane irradiated with UV at 800 mJ/cm² was easy to handle as a membrane itself but was nearly transparent. Because the membrane was slightly hard and had somewhat low flexibility, the membrane was less likely to fit uneven surfaces. In addition, when an attempt was made to place the membrane on an applicator, the membrane sometimes bounced back as if repelled and could not be placed on the applicator. The water content appeared to be somewhat low. For middle ear mucosal regeneration, the 0.7 mg/cm² vitrigel membrane with a grasping portion without UV irradiation was preferable, and the 1.4 mg/cm² vitrigel membrane with a grasping portion without UV irradiation was more preferable. It was confirmed that, for tympanic membrane regeneration, a three-times-thick membrane of 4.2 mg/cm² was preferable.

### INDUSTRIAL APPLICABILITY

According to the present invention, it is possible to provide a medical material that is easy to produce and has excellent handling performance.

### REFERENCE SIGNS LIST

100 Medical material
101 Slit
102 Grasping portion
103 Base portion

## Claims

1. A medical material comprising a hydrogel sheet that includes:
a base portion;
one or
an openable and closable grasping portion; and
a slit for separating a part of the grasping portion from the base portion.

2. The medical material according to Claim 1, wherein the grasping portion is openable and closable in a direction intersecting a sheet surface of the hydrogel sheet.

3. The medical material according to Claim 1, wherein the grasping portion is surrounded by the base portion.

4. The medical material according to Claim 1, wherein, in a cross-sectional view of the sheet taken along a direction in which the grasping portion extends from the base portion, the slit is formed obliquely with respect to the sheet surface.

5. The medical material according to Claim 1, wherein the medical material is for use in membrane regeneration, mucosal regeneration, or epithelial regeneration.

6. The medical material according to Claim 1, wherein the slit has a U-shape.

7. The medical material according to Claim 1, wherein a weight per unit area of the hydrogel sheet is 0.1 mg/cm² to 50 mg/cm².
